# EUROPEAN PATENT APPLICATION

(11) **EP 3 395 270 A1**
(43) Date of publication of application: **31.10.2018**
(21) Application number: 16879048.3
(22) Date of filing: 26.12.2016
(51) Int. Cl.: A61B 17/22, A61M 25/10

(54) **ENDOSCOPE TREATMENT INSTRUMENT**

(30) Priority: 24.12.2015 JP 2015251705
(71) Applicant: Zeon Corporation, Tokyo 100-8246 (JP)
(72) Inventor: KAWAJIRI, Koji, Tokyo 100-8246 (JP)
(74) Representative: Maiwald Patent- und Rechtsanwaltsgesellschaft mbH
(86) International application number: PCT/JP2016/088729
(87) International publication number: WO 2017/111164

(57) **Abstract**

Provided is an endoscope treatment instrument that includes a catheter tube (5) at a distal end of which a balloon section (2) is provided. At a distal end of a balloon membrane (3) constituting the balloon section (2), a distal end surface, which is continued to an outer surface (3a) of the balloon membrane (3), is bonded to a distal-end first outer peripheral surface (5a) of the catheter tube (5). At a proximal end of the balloon membrane (3), a proximal end surface, which is continued to an inner surface (3b) of the balloon membrane (3), is bonded to a distal-end second peripheral surface (5b) of the catheter tube (5), the distal-end second peripheral surface (5b) being located closer to the proximal end side than the distal-end first outer peripheral surface (5a) of the catheter tube (5). Due to this configuration, the balloon section can be withdrawn without being damaged when the endoscope treatment instrument is withdrawn through an endoscope.

## Description

### TECHNICAL FIELD

The present invention relates to an endoscope treatment instrument having a balloon part such as a balloon catheter for removing calculus used for example to remove the calculus formed in a bile duct or so.

### BACKGROUND ART

Several methods are known as a method for removing the calculus formed in the bile duct, that is the gallstone, from the body, and as one of those, the method of using the balloon catheter is known. When removing the gallstone from the bile duct using the balloon catheter, first the balloon catheter which the balloon part is deflated is inserted into the bile duct via an endoscope, then the balloon part is positioned further deeper than a position of the gallstone which is to be removed. Next, the balloon part is inflated then the balloon catheter is pulled back, thereby the gallstone is scraped out by the balloon part and is discharged out of the bile duct.

As such balloon catheter for removing calculus used for removing the gallstone, for example a structure described in the patent document 1 is known. As the balloon catheter shown in this patent document 1, a balloon membrane made of elastic material to form a balloon part is connected at a tip part (distal end part) of the catheter tube, and by introducing the fluid to the inside of this balloon membrane, the balloon membrane can be inflated.

For a conventional balloon catheter for removing the calculus having such balloon membrane, after using the balloon membrane by inflating it, the balloon membrane is deflated, then the balloon catheter needs to be removed from the body through a channel of the endoscope. When doing so, for the conventional balloon catheter, a force acting to tear up the connecting part of the distal end of the balloon membrane from the outer circumference face of the catheter tube is generated due to the friction between an inner wall of the channel and the balloon membrane.

Therefore, when removing the balloon catheter for removing calculus through the endoscope, in some cases, the balloon part was damaged at the connecting part of the distal end of the balloon membrane.

### PRIOR ART

[Patent document 1] JP Utility Model Application No.5-63551

### SUMMARY OF INVENTION

### PROBLEM TO BE SOLVED BY THE INVENTION

The present invention was attained in view of such circumstances, and the object is to provide the endoscope treatment instrument which allows easy removal of the balloon part without damaging the balloon part when removing the endoscope treatment instrument having the balloon part through the endoscope.

### MEANS FOR SOLVING THE PROBLEM

In order to attain the above objects, the endoscope treatment instrument according to the present invention has a catheter tube formed with a lumen along a longitudinal direction, and
a balloon part provided at a distal end part of the catheter tube, wherein
a distal end surface continuous with an outer surface of a balloon membrane constituting the balloon part is connected with a distal end side first outer circumference face of the catheter tube at a distal end of the balloon membrane, and

a proximal end surface continuous with an inner surface of the balloon membrane is connected with a distal end side second outer circumference face of the catheter tube positioned at a proximal end side than the distal end side first outer circumference face of the catheter tube at a proximal end of the balloon membrane.

The endoscope treatment instrument according to the present invention deflates the balloon membrane after using it by inflating it, and when removing the balloon catheter out of the body through the endoscope, the force tearing up the connecting part of the distal end of the balloon membrane will not act. The balloon membrane is subjected to just a force stretching it. Therefore, when removing the endoscope treatment instrument through the endoscope, it can be removed without damaging the balloon part.

Preferably, at the distal end part of the catheter tube, a distal end tip part is present at a position further distal end side than the distal end of the balloon membrane. By having such constitution, during the production steps of the endoscope instrument treatment, the distal end surface continuous with the outer side surface of the balloon membrane can be easily connected to the outer circumference face of the catheter tube. Further, as a result, when the balloon membrane is inflated, a distal end connecting part of the balloon membrane is effectively prevented from being peeled off.

The endoscope treatment instrument according to the present invention may further have a first cap member positioned at the distal end of the balloon membrane connected with the distal end side first outer circumference face. By having the first cap member, the strength of the distal end connecting part of the balloon membrane can be improved.

The endoscope treatment instrument according to the present invention may further have a second cap member positioned at the proximal end of the balloon membrane connected with the distal end side second outer circumference face. By having the second cap member, the strength of a proximal end connecting part of the balloon membrane can be improved.

As one embodiment of the present invention, a distal side wire insertion hole connected to the lumen of the catheter tube may be formed at the distal end of the catheter tube,
a proximal side wire insertion hole connected to the lumen may be formed at further proximal side than the balloon part along a longitudinal direction of the catheter tube,
the lumen heading towards a proximal end direction from the proximal side wire insertion hole may be closed by a cured filling,
the lumen heading towards a distal end direction from the proximal side wire insertion hole can be used as a distal side wire passage capable of inserting a wire, and
the filling may be formed with a slope so that the wire can be easily guided to the proximal side wire insertion hole from the distal side wire passage.

By having such constitution, the proximal end side of the guide wire only needs to be pulled out from the endoscope by a length slightly longer than a length between the distal side wire insertion hole to the proximal side wire insertion hole of the catheter tube.

As a result, the procedure of inserting the endoscope treatment instrument from the proximal end side of the guide wire, and the procedure of removing the endoscope treatment instrument can be done easily, hence the handling property improves and the endoscope treatment instrument can be easily exchanged. Further, the length of the guide wire pulled out from the endoscope can be shortened, thus sanitary management is also easy.

Further, the slope is formed to the cured filling in order to easily guide the wire towards the proximal side wire insertion hole from the distal side wire passage, thus just by pushing the proximal end of the guide wire from the distal side wire insertion hole, the proximal side of the guide wire is guided towards the proximal side wire insertion hole through the distal side wire insertion passage, and the guide wire can be easily pulled out from there. Also, since the cured fulling is filled in the lumen positioned near the proximal side wire insertion hole, the area near the proximal side wire insertion hole is reinforced; hence the kinking of this part is effectively prevented.

Note that, a stylet may be inserted in a removable manner at the lumen positioned further proximal side than the proximal end of the cured filling. By inserting the stylet, the rigidity of the proximal end part of the catheter tube can be increased, and an insertion characteristic of the endoscope treatment instrument along the guide wire is improved.

At the outer circumference face of the catheter tube positioned at the proximal end side of the proximal side wire insertion hole, a filling introducing hole to fill a fluid state filling prior to the curing into the lumen is formed, and the filling introducing hole may be closed by the cured filling. By having such constitution, the filling can be easily filled to the inside of the lumen of the catheter tube near the proximal side wire insertion hole. Also, since the filling introducing hole is closed by the cured filling, the filling introducing hole is not connected with the inside of the lumen.

Also, the edge of the opening of the filling introducing hole will be reinforced by part of the cured filling. Further, the edge of the opening of the proximal side wire insertion hole is also reinforced by part of the cured filling.

### BRIEF DESCRIPTION OF DRAWING

[Fig.1] Fig.1 is an entire figure of the balloon catheter for removing calculus according to one embodiment of the present invention.
[Fig.2A] Fig.2A is an enlarged partial cross section showing the distal end part of the catheter tube of the balloon catheter for removing calculus shown in Fig.1.
[Fig.2B] Fig.2B is an enlarged partial cross section showing the distal end part of the catheter tube of the balloon catheter for removing calculus according to other embodiment of the present invention.
[Fig.3] Fig.3 is an enlarged cross section along III-III line shown in Fig.2A.
[Fig.4] Fig.4 is an enlarged cross section along IV-IV line shown in Fig.1.
[Fig.5] Fig.5 is a partial cross section of the proximal end side wire insertion hole of the catheter tube shown in Fig.1.
[Fig.6A] Fig.6A is a schematic cross section showing the step of forming the balloon part of the balloon catheter for removing calculus shown in Fig.1.
[Fig.6B] Fig.6B is a schematic cross section showing the step of forming the balloon part of the balloon catheter for removing calculus shown in Fig.1.
[Fig.6C] Fig.6C is a schematic cross section showing the step of forming the balloon part of the balloon catheter for removing calculus shown in Fig.1.
[Fig.7A] Fig.7A is a partial schematic cross section showing the removal of the balloon catheter for removing calculus shown in Fig.1 through the endoscope.
[Fig.7B] Fig.7B is a partial schematic cross section showing the removal of the balloon catheter for removing calculus according to conventional example through the endoscope.

### DESCRIPTION OF EMBODIMENTS

Hereinafter, the present invention will be described based on the embodiments shown in the figures.

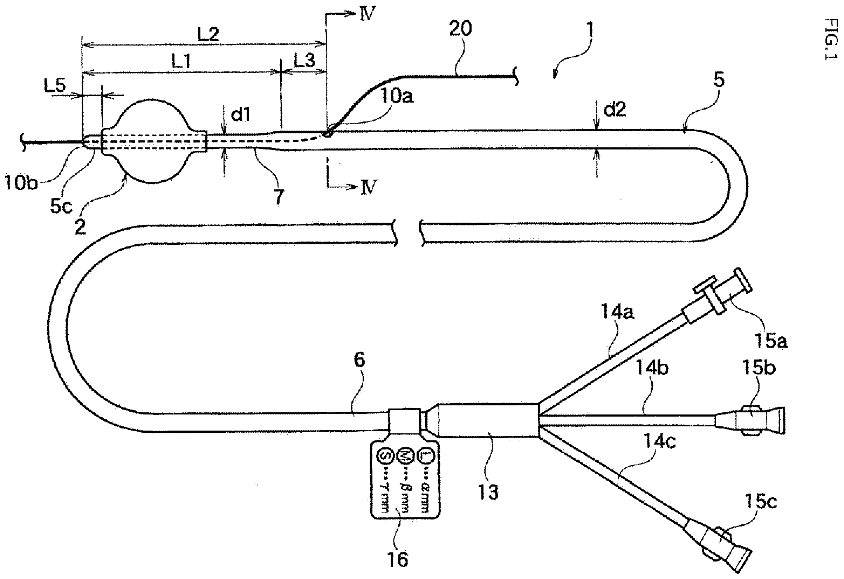

As shown in Fig.1, the balloon catheter 1 for removing calculus as the endoscope treatment instrument according to one embodiment of the present invention has a catheter tube 5, a balloon part (treatment part) 2, a cover 13, three branched tubes 14a to 14c, and three hubs 15a to 15c.

The catheter tube 5 of the balloon catheter 1 for removing calculus is a tube made of flexible material, and has a distal end part 7 which is an end part at a side inserted endoscopically into the body, and a proximal end part 6 positioned at the other end side. An outer diameter d2 of the proximal end part 6 of this catheter tube 5 is usually 1.0 to 4.2 mm, and an entire length is usually 500 to 2500 mm. Also, the material of the catheter tube 5 is not particularly limited as long as it has flexibility, and preferably it is polymer materials, and among polymer materials, polyamide resin or polyamide based elastomer is particularly preferable.

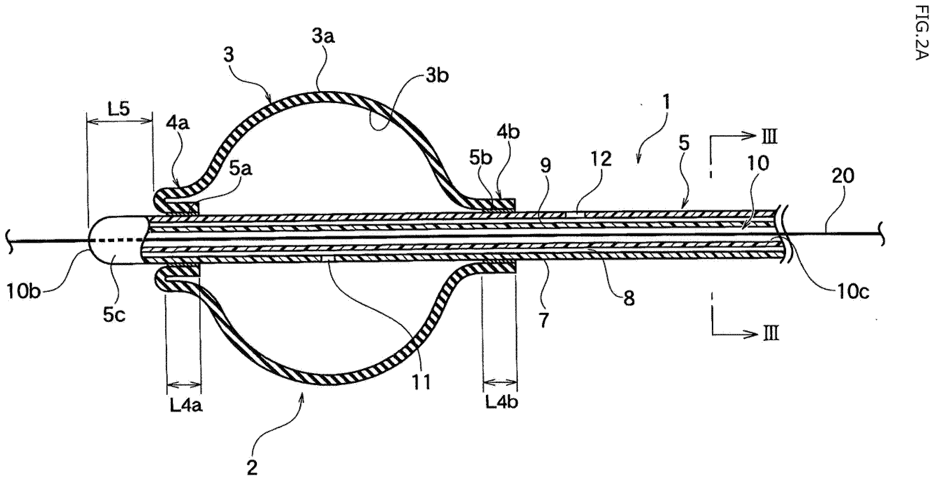

At the inside of the catheter tube 5, as shown in Fig.2A, a balloon lumen 8, a contrast agent lumen 9, and a main lumen 10 are formed. That is, the catheter tube 5 is constituted as a multi-lumen tube. The balloon lumen 8 is a lumen which is a flow passage for sending the fluid such as air used for inflating the balloon part 2 to the inside of the balloon membrane 3 constituting the balloon part 2, and the balloon lumen 8 runs through from the proximal end of the catheter tube 5 to the fluid discharge opening 11. The fluid discharge opening 11 is provided at the distal end part 7 of the catheter tube 5, and it is an opening positioned at the inside of the balloon membrane 3.

The contrast agent lumen 9 is the lumen used as the flow passage of the contrast agent when shooting X ray image for identifying the position of the calculus. This contrast agent lumen 9 runs through from the proximal end of the catheter tube 5 to the exhaustion opening 12 of the distal end part 7 of the catheter tube 5. The exhaustion opening 12 is an opening provided at the outside of the balloon part 2, and preferably provided to the position further proximal end side than the balloon part 2.

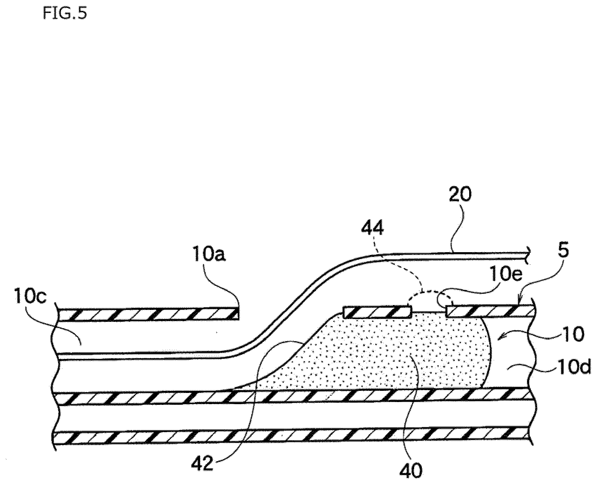

The main lumen 10 runs through from the proximal end to the distal end of the catheter tube 5; but it has a different function at the distal end side and the proximal end side across the proximal side wire insertion hole 10a shown in Fig.1. That is, the distal end opening of the main lumen 10 is formed at the distal end of the tube 5 positioned at the distal end side of the balloon part 2 as the distal end side wire insertion hole 10b, and the distal side main lumen 10c (see Fig.2A and Fig.5) positioned between the proximal end side wire insertion hole 10a and the distal end side wire insertion hole 10b functions as the wire lumen (distal end side wire passage).

Also, the proximal end side main lumen 10d of the main lumen 10 positioned at the proximal end side than the proximal end side wire insertion hole 10a shown in Fig.5 functions for example as the lumen for inserting the stylet or so. Note that, the stylet increases the rigidity of the catheter tube 5, and thus it is used to improve the insertion property against the endoscope and the body of the balloon catheter 1 for removing the calculus. For example, the stylet is a filament form body (stranded wire, straight metal line and so on) or a rod form body constituted by a metal such as stainless steel or so.

As shown in Fig.1, the proximal end side wire insertion hole 10a is formed at the proximal end side than the balloon part 2 along a longitudinal direction of the catheter tube 5, and also at the position having the outer diameter d2 at further proximal end side than the distal end part 7 having a small diameter. The length L2 between the proximal end side wire insertion hole 10a and the distal end side wire insertion hole 10b is longer than the longitudinal length L1 of the distal end part 7 formed in a small diameter, and preferably it is 35 to 800 mm. Also, the length L2 - L1 = L3 is preferably 5 to 400 mm.

As shown in Fig.5, the lumen 10 towards the proximal end direction from the proximal end side wire insertion hole 10a is closed by the cured filling 40, and the main lumen 10 is separated into the distal end side main lumen 10c and the proximal end side main lumen 10d by the filling 40, thereby these do not connect with each other. The distal end side main lumen 10c towards the distal end direction from the proximal end side wire insertion hole 10a can be used as the distal end side wire passage capable of inserting the wire 20. The cured filling 40 is formed with the inclined face 42 so that the wire can be easily guided towards the distal end side main lumen 10c from the proximal end side wire insertion hole 10a. The inclined face 42 allows easy guiding of the wire 20 towards the proximal end side wire insertion hole 10a from the distal end side main lumen 10c.

In order to attain the structure as shown in Fig.5, for example, first the proximal end side wire insertion hole 10a connecting only to the main lumen 10 at the predetermined position (the position L2 shown in Fig.1) of the longitudinal direction of the catheter tube 5 is formed. The inner diameter of the insertion hole 10a is about the same as the outer diameter of the temporary tube (temporary core member) which is not shown in the figure, and it is about the size which allows the temporary tube to enter inside of the insertion hole 10a by closely contacting thereto.

Also, at the same time, the filling introducing hole 10e connecting only to the main lumen 10 is formed at the position spaced apart a predetermined distance from the proximal end side wire insertion hole 10a to the proximal end direction. The inner diameter of the filling introducing hole 10e is not particularly limited as long as it has a size that the pre-cured fluid filling (the filling 40 in Fig.5) can be filled therefrom.

The pre-cured filling is not particularly limited as long as it is a filling which can be cured after injection, and for example ultraviolet ray curable resins such as acrylate based ultraviolet curable resin and epoxy based ultraviolet ray curable resin; heat curable resins such as epoxy based heat curable resin, phenol based heat curable resin, and polyester based heat curable resin or so; two components normal temperature curable resins such as epoxy based two components normal temperature curable resin, and acrylic based two components normal temperature curable resin or so; volatile solvent type adhesives such as vinyl acetate based volatile solvent based adhesive or so; and moisture curing adhesives such as cyanoacrylate based moisture curing adhesive or so may be mentioned.

The temporary tube is inserted into the proximal end side wire insertion hole 10a so that the distal end of the temporary tube not shown in the figure is positioned at the distal end side main lumen 10c of the main lumen 10, and the proximal end projects out from the proximal end side wire insertion hole 10a. Then, the pre-cured fluid filling is filled to the proximal end side than the temporary tube of the main lumen 10 from the filling introducing hole 10e. Note that, as the temporary core member inserted in the main lumen 10, the temporary tube having a tube form is used, but the temporary core member may be a solid form.

In the present embodiment, the temporary tube used as the temporary core member is constituted by a short tube having a uniform outer diameter and inner diameter along a longitudinal direction with excellent flexibility, and for example it is constituted by fluororesins such as polytetrafluoroethylene or so; polyamide resins, polyamide based elastomers, polyolefin based resins, and polyvinyl chloride resin or so. Preferably, the temporary core member is different from the catheter tube 5, and it is preferably a material which does not adhere with the pre-cured filling and with the cured filling 40 shown in Fig.5. From this point, the temporary core member is preferably constituted by fluororesins such as polytetrafluoroethylene or so, polyolefin based resins or so.

The amount of the pre-cured fluid filling to be filled is not particularly limited, and preferably it is about an amount sufficient to completely close the main lumen 10 at least from the proximal end side outer circumference face of the temporary tube inserted in the main lumen 10 towards the proximal end direction. Also, as shown in Fig.5, preferably the filling amount is about an amount which completely close the filling introducing hole 10e and slightly protruding out from there. Also, it may be an amount which slightly protrudes out of the main lumen 10 from the space between the proximal end side wire insertion hole 10a and the temporary tube.

Next, the filling 40 is cured by the curing method selected depending on the type of the pre-cured filling used, thereby the cured filling 40 closes the main lumen 10 from the proximal end side wire insertion hole 10a towards the proximal end direction. Then, the temporary tube is pulled out from the proximal end side wire insertion hole 10a while leaving the cured filling 40, thereby the temporary tube is removed from the main lumen 10. As a result, at the distal end side of the filling 40, the inclined face 42 inclined towards the distal end direction of the main lumen 10 from the proximal end side wire insertion hole 10a is formed by transferring along the outer circumference face of the temporary tube.

Note that, the protrusion part 44 of the filling 40 protruding from the filling introducing hole 10e may be removed, but it may be left as well. Also, the protrusion part 44 of the filling 40 adhered to the edge of the opening of the proximal end side wire insertion hole 10a may be left in order to protect the edge of the opening of the wire insertion hole 10a.

According to the present embodiment, the balloon catheter 1 for removing calculus having excellent handling property can be produced without providing a tube separate from the catheter tube 5 in the distal end side main lumen 10c used as the distal end side wire passage.

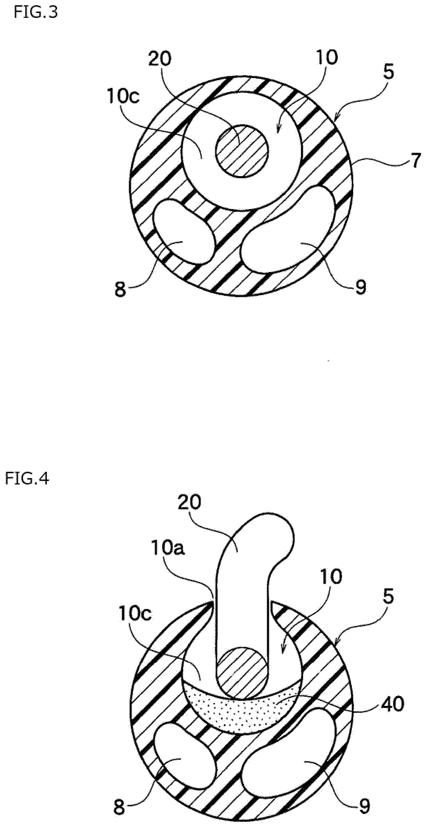

The cross section shape of the balloon lumen 8, the contrast agent lumen 9, and the main lumen 10 shown in Fig.3 and Fig.4 are not particularly limited, and these may take shapes which can be efficiently placed in the catheter tube 5. Note that, as the main lumen 10, the cross section shape is preferably circular shape which is same as the cross section of the general guide wire 20. Also, the cross section area of the balloon lumen 8 is preferably 0.03 to 0.5 mm², the cross section area of the contrast agent lumen 9 is preferably 0.08 to 1.0 mm², and the cross section area of the main lumen 10 is preferably 0.5 to 2.0 mm².

As shown in Fig.2A, the balloon membrane 3 constituting the balloon part 2 of the balloon catheter 1 for removing calculus is attached to the distal end part of the catheter tube 5 such that the balloon membrane 3 covers the fluid discharge opening 11. This balloon membrane 3 is formed by an elastic material, and inflates by introducing the fluid to the inside via the balloon lumen 8 of the catheter tube 5. Using this inflated balloon membrane 3, the calculus is scraped out or pushed out and the calculus in the body can be removed.

The elastic material forming the balloon membrane 3 preferably has 100% modulus (the value measured according to JIS K 6251) of 0.1 to 10 MPa, and particularly preferably 1 to 5 MPa. Also, as the specific example of suitable elastic material for forming the balloon membrane 3, natural rubbers, silicone rubbers, and polyurethane elastomers or so may be mentioned.

As shown in Fig.2A, the balloon membrane 3 constituting the balloon part 2 is a tubular form as a whole, and at both end parts, the first connecting part 4a and the second connecting part 4b are formed which connect with the outer circumference face of the catheter tube 5. An inflating part which inflates as the fluid is introduced to the inside is formed between these connecting parts 4a and 4b. In the present embodiment, the inflating part is formed so that the vertical cross section of the inflating part has an approximately circular shape when the fluid is not introduced into the inflating part. Due to such shape of the inflating part, the inflating part can be inflated larger than in case of using the balloon part having the balloon membrane of a cylinder shape as a whole, hence the removal performance of calculus or so using the balloon part 2 is improved.

Also, in the present embodiment, at the first connecting part 4a positioned at the distal end of the balloon membrane 3, the distal end surface continuous with the outer side surface 3a of the balloon membrane 3 is folded over and connects to the distal end side first outer circumference face 5a of the catheter tube 5. Also, at the second connecting part 4b positioned at the proximal end of the balloon membrane 3, the proximal end surface continuous with the inner side surface 3b of the balloon membrane 3 is connected, without being folded over, to the distal end side second outer circumference surface 5b of the catheter tube 5 positioned at further proximal end side than the distal end side first outer circumference face 5a of the catheter tube 5.

The method of connection at the first connecting part 4a and the second connecting part 4b is not particularly limited, and for example an adhesion using the adhesives, a thermal bonding, a welding using a solvent, and an ultrasonic welding or so may be mentioned. Also, the axial direction length L4a and L4b of each of connecting parts 4a and 4b may be same or different, and preferably it is within the range of 0.2 to 5 mm.

At the distal end part of the catheter tube 5, the first connecting part 4a of the balloon membrane 3 is connected to the outer circumference of the catheter tube 5 such that the distal end tip part 5c positioned further distal end side than the distal end of the balloon membrane 3 is left. The axial direction length L5 of the distal end tip part 5c is preferably 0.5 to 20 mm. By providing such distal end tip part 5c, during the production steps of the balloon catheter 1, a sufficient margin for connection can be taken at the catheter tube 5 side when connecting the distal end surface continuous with the outer side surface 3a of the balloon membrane 3 to the distal end side first outer circumference face 5a of the catheter tube 5, hence the step of connecting becomes easy, and a connection can be done easier in a good condition. As a result, the first connecting part 4a can have good connecting strength, thus the distal end connecting part (the first connecting part 4a) of the balloon membrane 3 being peeled off can be prevented effectively when the balloon membrane 3 is inflated.

The length of the balloon membrane 3 of the balloon part 2 (the length along the longitudinal direction of the catheter tube 5) is preferably 5 to 20 mm, and the thickness is preferably 0.10 to 0.50 mm.

For the balloon catheter 1 for removing calculus, when an exhaustion opening is provided for discharging the contrast agent, it is preferably provided to the surface of the catheter tube 5 at the position within 10 mm (more preferably within 5 mm) to the proximal end side from the proximal end of the second connecting part 4b of the balloon membrane 3. By providing the exhaustion opening 12 to this position, when the contrast agent is discharged from the exhaustion opening 12 after closing the body lumen by inflating the balloon membrane 3, the image of the body lumen positioned at the handling side than the inflated balloon part 2 can be taken efficiently.

Note that, the exhaustion opening may be provided at the surface of the catheter tube 5 at the position within 10 mm (more preferably within 5 mm) towards the distal end side from the distal end of the balloon part 2. In case the exhaustion opening is provided to the distal end side than balloon part 2, the contrast agent is discharged from the exhaustion opening before inflating the balloon part 2 in the body lumen to take an image of the body lumen and verify the condition inside the body lumen, thereby the balloon part 2 can be easily inserted to further appropriate position.

The shape of the connecting parts 4a and 4b positioned at both end sides of the balloon membrane 3 is not particularly limited, but preferably it is a cylindrical shape. In case the connecting parts 4a and 4b of the balloon membrane 3 have the cylindrical shapes, the inner diameters thereof are preferably about the same as the outer diameter of the catheter tube 5, and the length is preferably 0.5 to 5 mm.

The method of producing the balloon membrane 3 having the above mentioned shape is not particularly limited, and the known method as the method of producing the elastic material may be used, and preferably a dipping method is preferable. As the dipping method, a solution or a suspension is formed of which the elastic material and various additives depending on needs are dissolved in a solvent, and the mold having almost the same shape as the shape of the desired balloon membrane 3 is immersed in this solution (suspension) to coat the surface of the mold by the solution (suspension), and the solvent is evaporated to form a coating on the surface of the mold. This immersion and drying is repeated to produce the balloon membrane 3 having the desired thickness. Note that, depending on the type of the elastic material, a crosslinking is carried out after producing the coating.

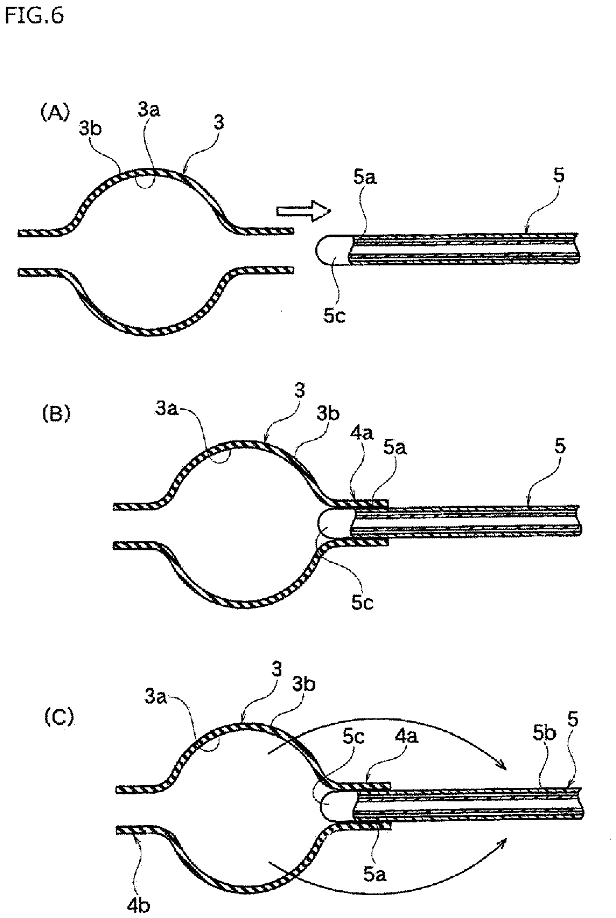

As shown in Fig.2A, the method for connecting the distal end side first outer circumference face 5a of the catheter tube 5 at the first connecting part 4a of the balloon membrane 3 by folding the distal end part of the balloon membrane 3 is not particularly limited, and for example the method shown in Fig.6A to Fig.6C may be mentioned. First, as shown in Fig.6A, the balloon membrane 3 which is turned inside out is prepared. That is, the outer side surface 3a of the balloon membrane 3 which will be the outer surface of the balloon part 2 of the finished balloon catheter 1 for removing calculus is faced inside, and the inner side surface 3b of the balloon membrane 3 which will be the inner surface of the balloon part 2 of the finished balloon catheter 1 for removing calculus is faced outside.

Then, as shown in Fig.6B, the distal end tip part 5c of the catheter tube 5 is passed to a tubular portion which will be the first connecting part 4a of the balloon membrane 3, and the tubular portion which will be the first connecting part 4a of the balloon membrane 3 is connected at the distal end side first outer circumference face 5a of the catheter tube 5 positioned at the proximal end side of the distal end tip part 5c. Next, as shown in Fig.6C, the balloon membrane 3 is folded over at the first connecting part 4a of the balloon membrane 3 so as to turn the balloon membrane 3 inside out, and the inner side surface 3b positioned at the second connecting part 4b of the balloon membrane 3 is connected to the distal end side second outer circumference face 5b of the catheter tube 5. As a result, the balloon part 2 having the constitution as shown in Fig.2A can be obtained.

For the balloon catheter 1 for removing calculus, the distal end part 7 of the catheter tube 5 attached to the balloon part 2 preferably is formed to have a smaller diameter part where the outer diameter is smaller than other part (the proximal end part 6) of the catheter tube 5, as shown in Fig.1. The reason for this is because by making the vertical cross section of the inflating part of the balloon membrane 3 to an approximately circular shape, the balloon catheter 1 for removing calculus can inflate the balloon sufficiently large, even if the part of the catheter tube 5 where the balloon part 2 is attached (the distal end part 7) has a smaller diameter. Furthermore, the distal end part 7 attains flexibility by having smaller diameter while maintaining the rigidity of the proximal end part 6 of the catheter tube 5 to certain degree, thereby the balloon catheter 1 for removing calculus can improve the handling property. In such case, the outer diameter d1 of the distal end part 7 of the catheter tube 5 is 50 to 95% of the outer diameter d2 of the proximal end part 6, and more preferably 60 to 90%.

The method of making the distal end part 7 of the catheter tube 5 smaller than the proximal end part 6 is not particularly limited, and the catheter tube 5 at the boundary between the distal end part 7 and the proximal end par 6 preferably has a taper form which becomes narrower towards the distal end. Also, for other method of making the distal end part 7 to have smaller diameter than the proximal end part 6, the method of providing a step between the distal end part 7 and the proximal end part 6 may be mentioned. The length L1 in the longitudinal direction of the distal end part 7 having a smaller diameter is preferably 30 to 400 mm.

The branch tubes 14a to 14c of the balloon catheter 1 for removing calculus are tubes connected to each lumen so that the procedures of sending the fluid to the balloon lumen 8 of the catheter tube 5, the procedure of introducing the contrast agent to the contrast agent lumen 9, and the procedure of inserting the stylet to the proximal end side of the main lumen 10 become easy.

The material of the branch tubes 14a to 14c is not particularly limited, and polymer material is preferably used. Also, the method of connecting the branch tubes 14a to 14c with each tube of the catheter tube 5 is not particularly limited, and for example the distal end part of the branch tubes 14a to 14c are molded in a taper form, then the adhesive is coated to the outer circumference face, and the end part thereof are inserted into the lumen of the catheter tube 5, thereby the bonding may be done.

The hubs 15a to 15c of the balloon catheter 1 for removing calculus are the members connected to the proximal end side of the branch tubes 14a to 14c. For example, the hub 15a and the branch tube 14a are connected to the balloon lumen 8 shown in Fig.2A, and the balloon inflating fluid can be injected or discharged from the hub 15a. Also, the hub 15c and the branch tube 14c are connected to the contrast agent lumen 9 as shown in Fig.2A, and the contrast fluid can be injected or discharged from the hub 15c. The hub 15b and the branch tube 14b are connected to the proximal end side main lumen 10d shown in Fig.5, and the stylet can be introduced or discharged from the hub 15b. The material of the hubs 15a to 15c is not particularly limited, and transparent polymer may be preferably used.

The cover 13 of the balloon catheter 1 for removing calculus shown in Fig.1 is provided so as to cover the connecting part between the catheter tube 5 and the branch tubes 14a to 14c in order to reinforce and protect the connecting part. The shape of the cover 13 is not particularly limited, and usually it is a box shape or a tubular shape. The material of the cover 13 is not particularly limited, and polymer material is preferably used. Also, a heat-shrinking tube can be used as the cover 13.

At the distal end side of the cover 13, a tag 16 is attached to the outer circumference of the catheter tube 5. The tag 16 shows specific information of the balloon catheter 1 such as how large the balloon part 2 will inflate by certain amount of air.

Next, as example of using the balloon catheter 1 for removing calculus of the present embodiment will be described using an example of removing a gall stone from a bile duct.

First, the endoscope is inserted into the body, and the tip of the endoscope is positioned near an entrance of the bile duct (duodenal papilla). Next, depending on needs, a cannulation catheter or so is used to insert the guide wire 20 in the body through a channel of the endoscope, and the distal end of the guide wire 20 is guided into the bile duct. Here, the guide wire 20 having an appropriate length is prepared in advance so that the proximal end side portion of the guide wire 20 protrudes out from the endoscope by the length slightly longer than the length (the distance L2) between the proximal end side wire insertion hole 10a and the distal end side wire insertion hole 10b of the balloon catheter 1 for removing calculus. Next, depending on the needs, the stylet is inserted to the proximal end side main lumen 10d through the hub 15b and the branch tube 14b, and also the guide wire 20 is passed from the distal end side wire insertion hole 10b to the distal end side main lumen (wire lumen) 10c which is between the proximal end side wire insertion hole 10a and the distal end side wire insertion hole 10b. Then, while keeping the balloon part 2 deflated, the balloon catheter 1 for removing calculus is inserted in the body along the guide wire 20 through the channel of the endoscope from the distal end side of the catheter tube 5, and the distal end part of the catheter 1 is guided to the bile duct.

Next, the catheter 1 is pushed further into the bile duct, then air is sent using a syringe or so into the balloon membrane 3 of the balloon part 2 through the hub 15a, the branch tube 14a, and the balloon lumen 8, thereby the balloon part 2 is inflated.

Next, the contrast agent is sent to the exhaustion opening 12 using a syringe or so through the hub 15c, the branch tube 14c, and the contrast agent lumen 9, thereby the contrast agent is discharged, and the X ray image of the bile duct is shot, and the condition of the gall stone is observed. Then, the balloon part 2 is kept inflated, and the catheter 1 is pulled back, thereby the gall stone can be scraped out of the bile duct towards the duodenum papilla by the balloon part 2.

Here, the catheter 1 of the present embodiment has the vertical cross section of the inflating part of the balloon membrane 3 being approximately circular form, hence the balloon part 2 can be inflated sufficiently large, and a space is scarcely formed between the bile duct lumen wall and the balloon part 2, also the gall stone can be easily scraped out. Note that, the gall stone scraped out of the bile duct is usually naturally discharged out of the body. That is, according to the balloon catheter 1 of the present embodiment, the calculus such as a gall stone or so can be promptly and easily discharged out of the body using the balloon part 2.

Also, when the balloon catheter 1 needs to be changed with other endoscope treatment instrument such as other balloon catheter or so, then the distal end of the guide wire 20 is left inside of the body and only the balloon catheter 1 is pulled out of the body along the guide wire 20. At that time, the catheter 1 can be easily taken out because the guide wire 20 is passed inside of the distal end side main lumen 10 by a relatively short distance L2 between the proximal end side wire insertion hole 10a to the distal end side wire insertion hole 10b along the catheter tube 5, hence the catheter 1 can be easily taken out.

That is, the proximal end side of the guide wire 20 only needs to be pulled out from the endoscope by a length slightly longer than the length corresponding at least to the distal end side wire insertion hole 10b to the proximal end side wire insertion hole 10a of the catheter tube 5. As a result, not only the procedure of inserting the guide wire 20 to the distal end side main lumen 10c, but also the procedure of removing the balloon catheter 1 along the wire 20 can be easily done, thus the handling property improves. Also, the balloon catheter 1 can be easily exchanged to other endoscope treatment instrument. Further, the length of the guide wire 20 protruding out from the endoscope can be made short, thus the sanitary management is also easy.

Further, the balloon catheter 1 of other embodiment has the cured filling 40 which is formed with the inclined face 42 in order to easily guide the wire 20 towards the distal end direction of the main lumen 10 from the proximal end side wire insertion hole 10a, hence the proximal end of the guide wire 20 only needs to be pushed from the distal end side wire insertion hole 10b, thereby the proximal end of the guide wire 20 is guided to the proximal end side wire insertion hole 10a through the distal end side wire passage 10c, and the guide wire 20 can be easily pulled out therefrom.

Also, the filling 40 is filled in the main lumen 10 at the position near the proximal end side wire insertion hole 10a, thus the area around the proximal end side wire insertion hole 10a is reinforced thus the kinking around this portion is effectively prevented.

Further in the present embodiment, the stylet is inserted in a removable manner at the proximal end side main lumen 10d at the position further proximal end side than the proximal end of the temporary tube 30, thus the rigidity of the proximal end part of the catheter tube 5 is increased, and the insertion characteristic of the balloon catheter 1 along the guide wire 20 is improved.

Also, in the present embodiment, at the outer circumference face of the catheter tube 5 positioned at the proximal end side of the proximal end side wire insertion hole 10a, the filling introducing hole 10e is formed which is to fill the pre-cured fluid filling to the inside of the lumen 10, and the filling introducing hole 10e is closed after the filling is cured. By taking such constitution, the pre-cured filling can be easily filled to the inside of the main lumen 10 of the catheter tube 1 at the area near the proximal end side wire insertion hole 10a. Also, the filling introducing hole 10e is closed by the cured filling, hence the filling introducing hole 10e and the inside of the main lumen 10 will not be connected.

Also, as shown in Fig.5, the edge of the opening of the filling introducing hole 10e is reinforced by part of the cured filling 40. Further, the edge of the opening of the proximal end side wire insertion hole 10a is reinforced by part of the cured filling 40.

Further, in the present embodiment, there is no need to insert other tube than the catheter tube 5 to the distal end side main lumen 10c used as the distal end side wire passage. Therefore, the flexibility at the distal end part of the balloon catheter 1 can be improved. Thus, the insertion characteristic of the endoscope treatment instrument into the body is improved.

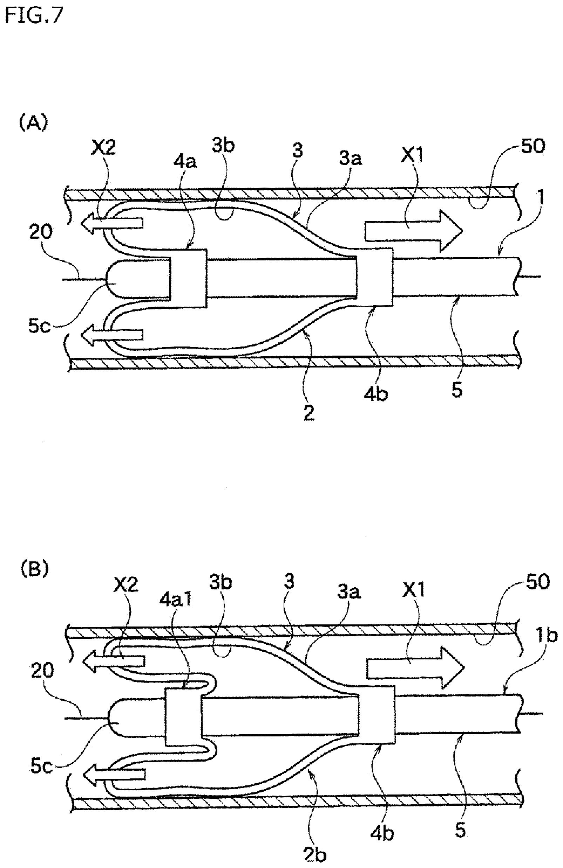

Particularly, there are no disadvantages in the present embodiment such as in case of the conventional examples when removing the balloon catheter 1 out of the body in X1 direction through a channel 50 of the endoscope as shown in Fig.7A by deflating the balloon membrane 3 after using the balloon membrane 3 by inflating it. That is, in the present embodiment, as shown in Fig.7A, even if a friction is generated as the deflated balloon membrane 3 contacts to the inner wall of the channel 50, the force tearing up the first connecting part 4a of the distal end of the balloon membrane 3 does not act on the first connecting part 4a of the distal end of the balloon membrane 3. The balloon membrane 3 is subjected to just a force stretching it in X2 direction. Therefore, when removing the balloon catheter 1 through the channel 50 of the endoscope, it can be removed without damaging the balloon part 2.

On the contrary to this, for the conventional balloon catheter 1b, as shown in Fig.7B, when the friction is generated as the deflated balloon membrane 3 contacts with the inner wall of the channel 50, the force tearing up the first connecting part 4a1 of the distal end of the balloon membrane 3 is acting. In the conventional balloon part 2, the inner side surface 3b of the balloon membrane 3 contacts with the outer circumference face of the catheter tube 5 at the first connecting part 4a1, hence the force pulling the balloon membrane 3 to X2 direction becomes the force rolling up the connecting part 4a1 of the balloon membrane 3, and the stress is concentrated to the boundary portion between the proximal end of the first connecting part 4a1 of the balloon membrane 3 and the non-connecting part adjacent thereto. As a result, the force tearing up the balloon membrane 3 acts on the boundary portion thereof. That is, in the conventional balloon catheter 1b, when removing the balloon catheter 1b through the channel 50 of the endoscope, the balloon part 2 may be damaged. Among these, in case of using the balloon membrane 3 of which the vertical cross section of the inflating part is an approximately circular shape, the loose portion exist in the balloon membrane 3 which the inflating part is not inflated, hence for the conventional balloon catheter 1b, the balloon part 2b is more likely to be damaged. However, the present embodiment does not have such disadvantages.

Further in the present embodiment, as shown in Fig.2A, the distal end tip part 5c is present at the position further distal end side than the distal end of the balloon membrane 3 at the distal end part of the catheter tube 5. By constituting as such, during the production steps of the balloon catheter 1, the step of connecting the distal end surface continuous with the outer side surface 3a of the balloon membrane 3 to the outer circumference face of the catheter tube 5 can be done easily. Thus, as a result, when the balloon membrane 3 is inflated, the first connecting part 4a of the distal end side of the balloon membrane 3 is effectively prevented from being peeled off.

### Second Embodiment

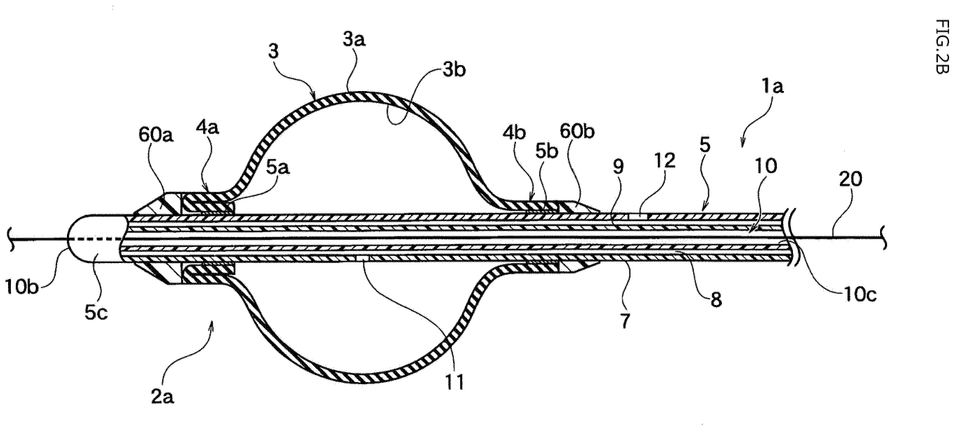

The balloon catheter 1a of the present embodiment shown in Fig.2B has the same constitutions and effects as the above mentioned embodiment except for the followings, and the description of the same parts are omitted. The same members are given with the same reference numbers. As shown in Fig.2B, the first taper member 60a is connected near the distal end tip part 5c of the catheter tube 5 in a manner that the first taper member 60a is adjacent to the distal end side of the distal end side first connecting part 4a of the balloon membrane 3 connected to the first outer circumference face 5a of the catheter tube 5. The first taper member 60a is a tubular member of which the outer shape is a taper form and becomes narrower towards the distal end side, and the thickness of the first taper member 60a at the proximal end is about the same as the thickness of the distal end side first connecting part 4a (the part where two membranes are stacked by folding over) of the balloon membrane 3.

Also, in the present embodiment, the second taper member 60b is connected near the distal end side second outer circumference face 5b of the catheter tube 5 in a manner that the second taper member 60b is adjacent to the proximal end side of the proximal end side second connecting part 4b of the balloon membrane 3 connected to the distal end side second outer circumference face 5b of the catheter tube 5. The second taper member 60b is a tubular member of which the outer shape is a taper form and becomes narrower towards the proximal end side, and the thickness of the second taper member 60b at the distal end is about the same as the thickness of the proximal end side first connecting part 4b of the balloon membrane 3.

The material of the first taper member 60a and the second taper member 60b is not particularly limited, and it is constituted by the same material as the catheter tube 5, but it does not necessarily have to be the same material.

By having the first taper member 60a and the second taper member 60b, the connecting part of the balloon membrane 3 is prevented from being peeled off which is caused when the balloon catheter 1a is inserted and removed through the endoscope and a forceps opening and inner wall face or so of the endoscope collides with a step present between the connecting part of the balloon membrane 3 and the catheter tube 5. Therefore, according to the balloon catheter 1a of the present embodiment, the chance of damaging the balloon part can be further reduced.

Note that, the present invention is not limited to the above mentioned embodiment, and various modifications can be done within the scope of the present invention.

For example, in the above mentioned embodiments, the balloon catheter 1 for removing calculus has one balloon, but it may have plurality of balloons. Also, the catheter tube 5 does not necessarily have to have the contrast agent lumen 9, and other lumen having different function other than already mentioned in above may be formed. Also the contrast agent lumen 9 may have other function than sending the contrast agent to the exhaustion opening 12. For example, a fluid such as saline solution or so may be send with great force to the contrast agent lumen 9 to discharge the fluid from the exhaustion opening 12, thereby it may function to push out the calculus by the fluid. In this case, the exhaustion opening 12 is formed diagonally with respect to the wall face of the catheter tube 5 so that the fluid is discharged diagonally to the proximal end side direction with respect to a center axis of the catheter tube.

Also, in the above mentioned embodiments, the catheter tube 5 has multi-lumen tube, but a catheter tube with a single lumen may be used. Also, other tube may be inserted at the inside of the catheter tube of the single lumen.

Also, in the above mentioned embodiments, the outer diameter of the distal end part 7 of the catheter tube 5 has a smaller diameter than the proximal end part, but it is not necessarily limited thereto, and for example the outer diameter of the distal end part and the proximal end part may be substantially the same.

Also, in the above mentioned embodiments, the balloon membrane 3 of the balloon part 2 inflated approximately rotationally symmetrically by taking the center axis of the catheter tube 5 as the axis of symmetry, but as disclosed in JP Patent Application Laid Open No.2008-194166, an eccentric inflating means may be provided to at least part of circumference direction of the balloon part 2 so that the balloon membrane 3 may inflate eccentrically with respect to the axis of the catheter tube 5.

Further, in the present embodiment, the endoscope treatment instrument was the balloon catheter for removing calculus which is used to remove a gall stone, but it is not limited thereto, and it may be a balloon catheter used for other purpose as long as it is used endoscopically.

### NUMERICAL REFERENCES

1...Balloon catheter for removing calculus (endoscope treatment instrument) 2...Balloon part (treatment part)
3...Balloon membrane
3a...Outer side surface
3b...Inner side surface
4a...First connecting part
4b...Second connecting part
5...Catheter tube
5a...Distal end side first outer circumference face
5b...Distal end side second outer circumference face
5c...Distal end tip part
6...Proximal end part
7...Distal end part
8...Balloon lumen
9...Contrast agent lumen
10...Main lumen
10a...Proximal end side wire insertion hole
10b...Distal end side wire insertion hole
10c...Distal end side main lumen
10d...Proximal end side main lumen
10e...Filling introducing hole
11...Fluid discharge opening
12...Exhaustion opening
13...Cover
14a to 14c...Branch tubes
15a to 15c...Hubs
20...Guide wire
40...Filling
50...Channel of endoscope
60a...First taper member
60b...Second taper member

## Claims

1. An endoscope treatment instrument comprising a catheter tube formed with a lumen along a longitudinal direction, and a balloon part provided at a distal end part of the catheter tube, wherein
a distal end surface continuous with an outer surface of a balloon membrane constituting the balloon part is connected with a distal end side first outer circumference face of the catheter tube at a distal end of the balloon membrane, and
a proximal end surface continuous with an inner surface of the balloon membrane is connected with a distal end side second outer circumference face of the catheter tube positioned at a proximal end side than the distal end side first outer circumference face of the catheter tube at a proximal end of the balloon membrane.

2. The endoscope treatment instrument according to claim 1 further comprising a distal end tip part of the catheter tube positioned at further distal end side than the distal end of the balloon.

3. The endoscope treatment instrument according to claim 1 or 2 further comprising a first cap member positioned at the distal end of the balloon membrane connected with the distal end side first outer circumference face.

4. The endoscope treatment instrument according to any one of claims 1 to 3 further comprising a second cap member positioned at the proximal end of the balloon membrane connected with the distal end side second outer circumference face.

5. The endoscope treatment instrument according to any one of claims 1 to 4, wherein
a distal side wire insertion hole connected to the lumen of the catheter tube is formed at the distal end of the catheter tube,
a proximal side wire insertion hole connected to the lumen is formed at further proximal side than the balloon part along a longitudinal direction of the catheter tube,
the lumen heading towards a proximal end direction from the proximal side wire insertion hole is closed by a cured filling,
the lumen heading towards a distal end direction from the proximal side wire insertion hole can be used as a distal side wire passage capable of inserting a wire, and
the filling is formed with a slope so that the wire can be easily guided to the proximal side wire insertion hole from the distal side wire passage.

6. The endoscope treatment instrument according to claim 5, wherein a filling introducing hole to fill a fluid state filling into the lumen prior to the curing is formed at an outer circumference face of the catheter tube position at the proximal end side of the proximal side wire insertion hole, and the filling introducing hole is closed by curing the fluid state filling.
